(19) Europäisches Patentamt
European Patent Office
Office européen des brevets

(11) Veröffentlichungsnummer: **0 205 602**
**B1**

(12) # EUROPÄISCHE PATENTSCHRIFT

(45) Veröffentlichungstag der Patentschrift: **04.07.90**

(51) Int. Cl.$^5$: **A 61 K 9/66**

(21) Anmeldenummer: **86900656.9**

(22) Anmeldetag: **06.12.85**

(86) Internationale Anmeldenummer:
**PCT/EP85/00679**

(87) Internationale Veröffentlichungsnummer:
**WO 86/03406 19.06.86 Gazette 86/13**

(54) **Verfahren zur Herstellung von Weichgelatinekapseln.**

(30) Priorität: **12.12.84 DE 3445237**

(43) Veröffentlichungstag der Anmeldung:
**30.12.86 Patentblatt 86/52**

(45) Bekanntmachung des Hinweises auf die Patenterteilung:
**04.07.90 Patentblatt 90/27**

(84) Benannte Vertragsstaaten:
**AT BE CH DE FR GB IT LI NL**

(56) Entgegenhaltungen:
**EP-A-0 143 857**
**DE-A-1 617 845**
**FR-A-2 261 752**

**"Die Kapsel" Wissenschaftliche Verlagsgesellschaft Stuttgart 1982**

(73) Patentinhaber: **R.P. Scherer GmbH**
**Gammelsbacher Strasse 2 Postfach 1243**
**D-6930 Eberbach/Baden (DE)**

(72) Erfinder: **BROX, Werner**
**Kätchen-Paulusstrasse 6**
**D-6124 Beerfelden (DE)**
Erfinder: **GABLER, Wilfried**
**Neckarstaden 18**
**D-6900 Heidelberg (DE)**

(74) Vertreter: **Werner, Hans-Karsten, Dr. et al**
**Deichmannhaus am Hauptbahnhof**
**D-5000 Köln 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 205 602 B1

**Beschreibung**

Gegenstand der vorliegenden Erfindung Weichgelatinekapseln mit einer Hülle aus Gelatine, mindestens einem Weichmacher und einer Kapselfüllung, die mindestens einen pharmakologischen Wirkstoff und ein Lösungsmittel enthält ist ein Verfahren zur Herstellung von.

Nachdem es in den 30iger Jahren gelungen war, Weichgelatinekapseln so herzustellen, daß die Kapselherstellung und -füllung in einem Arbeitsgang erfolgt, haben sich Gelatinekapseln, insbesondere Weichgelatinekapseln als Arzneiform immer stärker durchgesetzt. Sie weisen gegenüber anderen Darriechungsformen eine Reihe von Vorteilen auf. So sind sie geruch- und geschmacklos, lassen sich leicht einnehmen, und dank ihrer Quellfähigkeit und Wasserlöslichkeit werden die Arzneistoffe im Magen leicht freigesetzt. Zahlreiche Arzneistoffe, die sich sonst wegen ihrer Oxidations- und Lichtempfindlichkeit, Thermolabilität oder Hygroskopizität nicht zu anderen Arzneiformen verarbeiten lassen, können ohne Beeinträchtigung ihrer Wirkung verkapselt werden.

Weichgelatinekapseln dienen vorwiegend zur Aufnahme von Flüssigkeiten, und zwar öligen Lösungen, Suspensionen oder Emulsionen. Als Füllmaterialien sind pflanzliche, tierische und mineralische Öle, flüssige Kohlenwasserstoffe, ätherische Öle und auch Polyethylenglykole in Gebrauch. Zur Konsistenzerhöhung werden auch Fette und Wachse verwendet oder zugesetzt.

Polyethylenglykole zeichnen sich dabei gegenüber den anderen möglichen Füllmaterialien für Weichgelatinekapseln durch eine Reihe von Besonderheiten aus.

Im Gegensatz zu öligen Flüssigkeiten sind flüssige Polyethylenglykole mit Wasser unbegrenzt mischbar. Da Polyethylenglykole gleichzeitig viele in Wasser schwer- oder gar nicht lösliche Arzneistoffe zu lösen vermögen, ermöglicht die Verwendung von Polyethylenglykolen bei derartigen Arzneistoffen eine besonders vorteilhafte Wirkstoffreisetzung. Schwer wasserlösliche Arzneistoffe, die in Polyethylenglykolen gelöst und dann in Weichgelatinekapseln abgefüllt sind, zeichnen sich in vielen Fällen durch eine besonders gute Bioverfügbarkeit der Arzneistoffe aus.

Aus der DE—A—16 17 845 ist ein Depot-Arzneimittel in Kapselform bekannt, bei denen der Wirkstoff in fließfähiger Lösung oder Suspension zusammen mit physiologisch indifferenten Natur- und/oder Kunststoffen verkapselt werden. Zugleich sind geringe Mengen eines Wasser-Öl-Emulgier- oder Hilfslösungsmittels vorhanden.

Die FR—A—75 05457 offenbart ein Verfahren zur Erhöhung der Bioverfügbarkeit von Digoxin, wobei man eine Lösung von Digoxin in eine lösliche Kapselhülle bringt.

Die nicht vorpublizierte EP—A—0 143 857 offenbart eion Coronartherapeutikum in Form von Weichgelatinekapseln, die den Wirkstoff Nifedipin in einem organischen Lösungsmittel enthalten. Das Nifedipin wird mit Polyvinylpyrrolidon unter Verzicht auf Glycerin in Tetrahydrofurfurylalkohol-Polyethylen-Glycolether gelöst und somit ein geringeres Kapselgewicht ermöglicht.

Aus der DE—A—33 07 353 der Anmelderin sind Weichgelatinekapseln bekannt, bei denen die aufgetrocknete Hülle der Kapsel 4 bis 40 Gew.-% Sorbit und/oder Sorbitane enthält, das in der Kapselfüllung zur Lösung und Suspendierung des Wirkstoffs verwendete Polyethylenglykol zumindestens 50 Gew.-% eines Polyethylenglykols mit einem mittleren Molekulargewicht von 600 ist, und das Kapselfüllgut bis zu 20 Gew.-% Glycerin und/oder 1,2-Propylenglykol enthält. Diese Weichgelatinekapseln haben sich außerordentlich bewährt, jedoch weisen sie noch immer den Nachteil auf, daß eine Reihe von Wirkstoffen in Polyethylenglykol nicht ausreichend löslich sind. Dies hat zur Folge, daß zur Verkapselung solcher schwerlöslichen Wirkstoffe verhältnismäßig große Kapseln notwendig sind. Es besteht somit nach wie vor das Bedürfnis nach Lösungsmitteln, die größere Mengen schwerlöslicher Wirkstoffe zu lösen vermögen und dennoch zu stabilen Weichgelatinekapseln verarbeitet werden können. Die für Humanzwecke geeigneten Lösungsmittel wie Ethanol, Propylenglykol, Dimethylacetamid, Milchsäure, Glycerin, Butandiol und die Polyethylenglykolether des Tetrahydrofurfurylalkohols haben sich nach den Untersuchungen der Anmelderin als ungeeignet erwiesen, in größeren Mengen in Weichgelatinekapseln eingebracht zu werden, da die mit disen Lösungsmitteln hergestellten Kapselfüllungen bereits nach kurzer Zeit zur Erweichung und zur Verformung der hergestellten Kapseln führen und diese daher nicht handelsfähig sind. Die Ursache für diese Stabilitätsprobleme sind offensichtlich in der guten Wassermischbarkeit und dem niedrigen Molekulargewicht dieser Lösungsmittel zu suchen.

Es wurde jetzt überraschenderweise gefunden, daß Gemische von Polyethylenglykolethern des Tetrahydrofurfurols doch als Lösunsgmittel für Wirkstoffe in Weichgelatinekapseln geeignet sind, wenn man die Kapselfüllung in eine wasserhaltige Gelatinehülle einkapselt und die erhaltenen Kapseln mindestens 5 Tage, vorzugsweise 6 bis 10 Tage intensiv trocknet.

Gegenstand der vorliegenden Erfindung ist demnach ein Verfahren zur Herstellung von Weichgelatinekapseln mit einer Hülle aus Gelatine, mindestens einem Weichmacher und einer Kapselfüllung, die mindestens einen pharmakologischen Wirkstoff und ein Lösungsmittel enthält, dadurch gekennzeichnet, daß man als Lösungsmittel mindestens 50 Gew.-% eines Gemisches von Polyethylenglykolethern des Tetrahydrofurfurylalkohols der Formel I

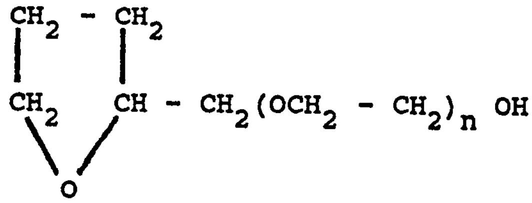

I

in der n = 1 bis 6 ist, verwendet, in eine wasserhaltige Gelatinehülle einkapselt und die erhaltenen Kapseln mindestens 5 Tage, vorzugsweise 6 bis 10 Tage, intensiv trocknet.

Nach 5 bis 10 Tagen sind offensichtlich die bei der Verkapslung aus der wasserhaltigen Gelatinehülle in die Kapselfüllung eingedrungenen Wassermengen so weitgehend entfernt worden, daß sie zusammen mit den Polyethylenglykolethern des Tetrahydrofurfurylalkohols nicht mehr in der Lage sind, die Gelatinehülle nachträglich wieder zu erweichen und das Austreten des niedermolekularen Lösungsmittels zu ermöglichen. Eine so lange und intensive Trocknung ist bei Weichgelatinekapseln ausgesprochen ungewöhnlich. Üblicherweise werden Weichgelatinekapseln nur 1 bis 4 Tage getrocknet. Längere Trocknungszeiten verursachen nicht nur unnötige Kosten, sondern führen auch zu einer unerwünschten Verhärtung und Versprödung der Kapselhülle. Bei dem erfindungsgemäßen Verfahren wandern offensichtlich gewisse Mengen der Polyethylenglykolether des Tetrahydrofurfurols in die Kapselhülle und wirken dort als Weichmacher, die der sonst beobachteten Verhärtung und Versprödung der Kapselhülle bei zu langem Trocknen entgegenwirken.

Diese als Weichmacher wirkenden Mengen der Polyethylenglykolether des Tetrahydrofurfurols in der Kapselhülle führen jedoch auch zu einer erhöhten Empfindlichkeit der erfindungsgemäßen Kapseln gegen Luftfeuchtigkeit, so daß die fertigen Kapseln unter Abschluß von Luftfeuchtigkeit gelagert werden sollen. Unter Abschluß von Luftfeuchtigkeit gelagerte erfindungsgemäß hergestellte Kapseln haben sich jedoch auch nach 3 Jahren noch als völlig stabil erwiesen. Es ist somit erstmals gelungen, ein niedermolekulares, mit Wasser in jedem Verhältnis mischbares organisches Lösungsmittel zu stabilen Weichgelatinekapseln zu verarbeiten und damit ein breites Feld von neuen Anwendungsmöglichkeiten dieses Lösungsmittels zu eröffnen.

Die Polyethylenglykolether des Tetrahydrofurfurols besitzen die allgemeine Formel I. Für Humanzwecke geeignet sind insbesondere Gemische in denen n = 1 bis 6 ist oder in denen n = 1 und 2 ist. Besonders geeignet sind somit die Handelsprodukte "Glycofurol 75" (eine Mischung des Monoethylenglykolethers und des Diethylenglykolethers im Verhältnis von ungefähr 1:1 und einem mittleren Molekulargewicht von ca. 168) sowie Tetraglycol® (Mischung aus Monoethylenglykolether, Diethylenglykolether und unterschiedlichen Anteilen an Tri-, Tetra-, und Pentaethylenglykolethern mit einem mittleren Molekulargewicht von ca. 190). In diesen Lösungsmitteln sind eine Reihe von schwerlöslichen Wirkstoffen wesentlich besser löslich als in Polyethylenglykolen, so daß sich erfindungsgemäß wesentlich kleinere und angenehmer einnehmbare Weichgelatinekapseln herstellen lassen. Beispielsweise sind die beiden interessanten Wirkstoffe Diazepam und Nifedipin in dem erfindungsgemäß verwendeten Lösungsmitteln praktisch doppelt so gut löslich wie in Polyethylenglykol 400.

Es wurde weiterhin gefunden, daß nicht nur die reinen Gemische von Polyethylenglykolethern des Tetrahydrofurfurylalkohols geeignet sind, sondern darüberhinaus auch Gemische mit anderen üblichen Lösungsmitteln und Lösungsvermittlern. Insbesondere Polyethylenglykole mit dem Molekulargewicht 300 bis 600 können bis zu 50 Gew.-% den Polyethylenglykolethern des Tetrahydrofurfurylalkohols zugesetzt werden, ohne dessen Löslichkeit für schwerlösliche Wirkstoffe zu stark zu verändern. Zur Erhöhung der Löslichkeit gewisser Wirkstoffe in den erfindungsgemäß verwendeten Lösungsmitteln und Lösungsmittelgemischen können diesen auch hierin lösliche Lösungsvermittler und Emulgatoren zugesetzt werden.

Der Gelatinehülle werden die üblichen Weichmacher wie Glycerol, Sorbit, Sorbitane usw. zugesetzt.

Der Kapselhülle können ferner andere übliche Hilfsstoffe wie Konservierungsmittel (z.B. p-Aminobenzosäureester und Kaliumsorbat) Farbstoffe, Farbpigmente, Geschmacksstoffe oder Aromastoffe zugesetzt werden. Gewünschtenfalls können die fertigen Kapseln auch nachträglich noch mit Spezialüberzügen versehen werden, die die Applikation erleichtern oder verbessern. Die erfindungsgemäß verwendeten Polyethylenglykolether des Tetrahydrofurfurylalkohols sind pharmakologisch und toxikologisch unbedenklich und werden bereits in erheblichem Umfang zur Herstellung von Injektionslösungen eingesetzt. Die orale Applikation von Wirkstoffen in diesen Lösungsmitteln ist bisher unterblieben, da die Polyethylenglykolether des Tetrahydrofurfurylalkohols ausgesprochen unangenehm schmecken. Es ist daher ein weiterer Vorteil der vorliegenden Erfindung, diese für viele Wirkstoffe guten Lösungsmitteln jetzt auch für orale Applikationsformen zugänglich gemacht zu haben. Die relativ geringen Mengen dieser Lösungsmittel in der Kapselhülle beeinträchtigen jedenfalls die orale Applikation der erfindungsgemäß hergestellten Weichgelatinekapseln nicht.

In den nachfolgenden Beispielen und Vergleichsversuchen werden die erfindungsgemäßen Weichgelatinekapseln und das Verfahren zu ihrer Herstellung näher erläutert.

Beispiel 1

Jeweils 500 mg des Handelsproduktes Tetraglykol® wurden zunächst ohne Zusatz von Hilfsstoffen oder Wirkstoffen in Weichgelatinekapseln eingebracht und die Kapseln zum Teil nur 1 Tag, zum Teil 3, 5, 8 und 10 Tage bei 20°C und 20 bis 25% relativer Luftfeuchtigkeit getrocknet. Aus den nur einen Tag getrockneten Kapseln trat nach einigen Wochen das Lösungsmittel durch die Kapselhülle nach außen aus, wodurch diese Kapseln als Handelsprodukt unbrauchbar wurden. Die Kapseln, die 3 Tage getrocknet wurden, waren nur 12 Monate stabil. Die Kapseln, die erfindungsgemäß 5, 8 oder 10 Tage getrocknet worden waren, waren unter gleichen Lagerbedinungen 3 Jahre völlig stabil. Auch bei 8 und 10 Tagen Trocknung war keine Versprödung eingetreten.

Beispiel 2

Vergleichende Löslichkeitsuntersuchungen der Wirkstoffe Nifedipin und Diazepam in Polyethylenglykol 400 und Tetraglykol® ergaben als maximale Löslichkeit bei 21°C in Gew.-% folgende Werte:

| Wirkstoff | Diazepam | Nifedipin |
|---|---|---|
| PEG 400 | 5,9% | 4,9% |
| Tetraglykol® | 11,5% | 10,2%. |

Entsprechend den üblichen Dosierungen von Nifedipin und Diazepam wurden folgende Rezepturen erstellt. Die Angaben betreffen mg/Kapsel.

| | | |
|---|---|---|
| 1. | Nifedipin | 10,0 mg |
| | Tetraglykol® | 110,0 mg |
| | | 120,0 mg |

| | | |
|---|---|---|
| 2. | Diazepam | 5,0 mg |
| | Tetraglykol® | 115,0 mg |
| | | 120,0 mg. |

| | | |
|---|---|---|
| 3. | Diazepam | 5,0 mg |
| | Tetraglykol® | 60,0 mg |
| | PEG 400 | 50,0 mg |
| | Glycerol | 5,0 mg |
| | | 120,0 mg |

Diese Wirkstofflösungen wurden in dem sehr kleinen und daher gut schluckbaren Kapselformat 2 minims oval verkapselt und jeweils 6 Tage getrocknet. Die Kapseln waren wie die Kapseln ohne Wirkstoff bei Lagerung ohne Zutritt von Luftfeuchtigkeit lagerstabil.

**Patentansprüche**

1. Verfahren zur Herstellung von Weichgelatinekapseln mit einer Hülle aus Gelatine, mindestens einem Weichmacher und einer Kapselfüllung, die mindestens einen pharmakologischen Wirkstoff und ein Lösungsmittel enthält, dadurch gekennzeichnet, daß man als Lösungsmittel mindestens 50 Gew.-% eines Gemisches von Polyethylenglykolethern des Tetrahydrofurfurylalkohols der Formel I

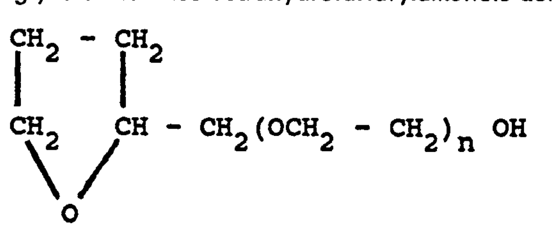

I,

in der n = 1 bis 6 ist, verwendet, in eine wasserhaltige Gelatinehülle einkapselt und die erhaltenen Kapseln mindestens 5 Tage intensiv trocknet.

2. Verfahren gemäß Anspruch 1, dadurch gekennzeichnet, daß 6 bis 10 Tage intensiv getrocknet wird.

3. Verfahren gemäß Anspruch 1 oder 2, dadurch gekennzeichnet, daß n = 1 und 2 ist.

**Revendications**

1. Procédé de production de capsules en gélatine molle comprenant une enveloppe en gélatine, au moins un émollient et un remplissage de capsule, qui contient au moins une substance pharmacologiquement active et un solvant, caractérisé en ce qu'on utilise comme solvant au moins 50% de poids d'un mélange d'éthers de polyéthylèneglycol de l'alcool tétrahydrofurfurylique de la formule I

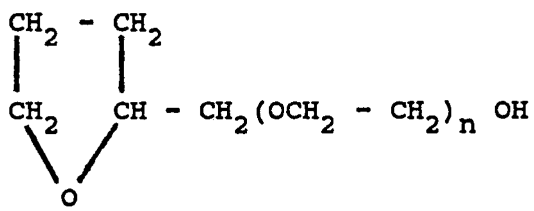

I,

dans laquelle n = 1 à 6, qui est encapsulé dans une enveloppe de gélatine contenant de l'eau et en ce que les capsules obtenues sont séchées intensivement pendant au moins 5 jours.

2. Procédé selon la revendication 1, caractérisé en ce qu'un séchage intensif a lieu pendant 6 à 10 jours.

3. Procédé selon la revendication 1 ou 2, caractérisé en ce que n = 1 et 2.

**Claims**

1. A process for preparing soft gelatin capsules comprising a gelatin sheath, at least one plasticizer and a capsule filling containing at least one pharmacologically active substance and a solvent, characterized in that as a solvent there is used at least 50% by weight of a mixture of polyethylene glycol ethers of tetrahydrofurfuryl alcohol having the formula I

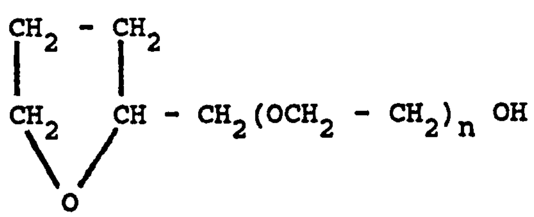

I,

wherein n = 1 to 6, encapsulated in a water-containing gelatin sheath, and that the capsules obtained are dried for at least 5 days.

2. The process according to claim 1, characterized in that an intensive drying is effected for 6 to 10 days.

3. The process according to claims 1 or 2, characterized in that n = 1 and 2.